# EUROPÄISCHE PATENTSCHRIFT

(11) **EP 3 803 319 B1**
(45) Veröffentlichungstag und Bekanntmachung des Hinweises auf die Patenterteilung: **13.09.2023**
(21) Anmeldenummer: 19725928.6
(22) Anmeldetag: 14.05.2019
(51) Int. Cl.: G01N 1/10, G01N 1/40, G01N 33/18, H01J 49/04

(54) **UNTERWASSER-GASMESSVORRICHTUNG FÜR IN WASSER GELÖSTE GASE**
UNDERWATER GAS MEASUREMENT APPARATUS FOR GASES DISSOLVED IN WATER
DISPOSITIF SUBMERSIBLE DE MESURE DES GAZ POUR DES GAZ DISSOUS DANS L'EAU

(30) Priorität: 24.05.2018 DE 102018112526
(43) Veröffentlichungstag der Anmeldung: 14.04.2021
(73) Patentinhaber: B2 Sensors GmbH, 24106 Kiel (DE)
(72) Erfinder: BENAVIDES NORIEGA, Roberto Enrique, 24106 Kiel (DE); BERGER, Axel, 24106 Kiel (DE)
(74) Vertreter: Puschmann Borchert Kaiser Klettner Patentanwälte Partnerschaft mbB
(86) Internationale Anmeldenummer: PCT/DE2019/100437
(87) Internationale Veröffentlichungsnummer: WO 2019/223835

(56) Entgegenhaltungen:
- WO-A1-2014/183133
- US-A- 4 662 826
- US-A1- 2002 079 442
- US-A1- 2009 084 976

## Beschreibung

Die Erfindung betrifft eine Unterwasser-Gasmessvorrichtung für in Wasser gelöste Gase einsetzbar auch in großen Wassertiefen, insbesondere in der Tiefsee-Wassersäule oder am Meeresboden.

Gattungsgemäße Vorrichtungen sind als wissenschaftliche Messgeräte in der Ozeanographie und marinen Geologie sowie als Kontrollgeräte zur Überwachung unterseeischer Installationen der Hydrokarbon-Wirtschaft, z.B. Pipelines und Bohrinseln, bekannt. In ihrer einfachsten Ausführung umfassen sie Probennehmer mit einer Mehrzahl von Probenflaschen (in der Regel 24-48 Niskin-Flaschen maximal), die einzeln zu verschiedenen Zeitpunkten befüllt und üblich druckversiegelt an die Oberfläche zurück verbracht werden. Die Messung der enthaltenen Gase erfolgt dann mittels Gaschromatografie in einem Labor, also ex situ. Die Laborauswertung ist dabei zeit- und kostenaufwendig.

Demgegenüber besteht großes Interesse an in situ Messungen mit hoher zeitlicher Auflösung und genauer Ortszuweisung, etwa um Gasverteilungen und Gasaustritte aus ozeanischen Quellen zu erfassen. Solche Daten können mit kompakten, unterwassertauglichen Messsystemen untersucht werden, die dazu an ein tauchfähiges Aggregat angebracht werden, etwa an einer Verankerung ("mooring") oder einem Ozean-Boden-Hydrophon oder an einem Tauchfahrzeug (bemannt oder ferngelenkt oder autonom). Beispielsweise wurde der Ausstoß von Methan aus seismisch kartierten Gashydratvorkommen bereits 1997 erstmals mit in situ Methansensoren montiert an Tauchbooten profiliert.

Die Messprinzipien der in situ Systeme können recht unterschiedlich sein. Frühe Sensoren basierten etwa auf der reversiblen Anlagerung von Methan an Zinndioxid unter Änderung des elektrischen Widerstandes oder auf der charakteristischen Absorption von Infrarot-Licht in Kohlendioxid. Heute stehen dank verbesserter Lichtquellen, Detektoren und Rechenkapazität mit kleinem Bauraum weiter entwickelte Techniken wie die Massen-Spektroskopie (MS), die Integrated Cavity Output Spectroscopy (ICOS) oder auch die Surface-Enhanced Raman Spectroscopy (SERS) zusätzlich zur Verfügung. Auch die gewöhnliche Laser-AbsorptionsSpektroskopie ist durch inzwischen verfügbare durchstimmbare und/oder auf "exotische" Wellenlängen abgestimmte Laser gereift und erlaubt die gleichzeitige Bestimmung mehrerer Gasspezies in einem Gasvolumen mit hoher Genauigkeit.

Einige typische Anforderungen an Unterwasser-Gasmessvorrichtungen für gelöste Gase kann man wie folgt zusammenfassen:
- Ansprechzeit T₉₀ höchstens wenige Sekunden, Abklingzeit möglichst nicht länger;
- keine Kreuzempfindlichkeiten des Sensors auf verschiedene, gleichzeitig anwesende Gase (z.B. ein typisches Problem bei verschiedenen Alkanen);
- keine oder zumindest kalibrierbare Abhängigkeiten von Druck, Temperatur und Feuchte;
- robust gegen Außendruck bis 60 MPa (6000 m Wassertiefe);
- möglichst geringer Energieverbrauch (wenn keine Kabelverbindung zum Schiff);
- eine sehr große Anzahl an in situ Messungen soll möglich sein.

Die vorgenannten Messprinzipien sind alle nur in der Gasphase anwendbar, d.h. das gelöste Gas muss in einem ersten Schritt stets aus der Wasserphase abgeschieden werden. Da die Gasmessvorrichtung zum Schutz vor dem Umgebungsdruck durch einen Druckbehälter, typisch einen Zylinder aus Titan oder Edelstahl, vollständig umschlossen sein muss, wird eine Gaseintrittsöffnung im Druckbehälter vorgesehen und mit einer semipermeablen, für Gas durchlässigen Membran wasserdicht verschlossen. Hinter der Membran wird zudem eine Stützeinrichtung für die Membran gegen hydrostatischen Druck angeordnet, beispielsweise eine perforierte oder poröse Metallplatte mit offenem Porenraum, die fest mit dem Druckbehälter verbunden, z.B. verschraubt, wird. Die Membran kann aus einem Kunststoffgewebe, z.B. Polyestervlies, bestehen und ist in der Regel mit Silikon hydrophob beschichtet. Der Druckbehälter ist somit bis zu hohen Drücken wasserdicht, aber an einer vorbestimmten Stelle nicht dicht gegen Gaseintritt.

Die Ausgestaltungsmöglichkeiten des Gaseinlasses in den Druckbehälter sind vielfältig, und es wird insbesondere auf die Druckschrift US 2009/0084976 A1 verwiesen, die dazu sehr umfangreiche Vorschläge macht. Den Kern der Druckschrift bildet ein u.a. tiefseetaugliches Massenspektrometer zur Analyse unterschiedlicher natürlicher Gase. Solche Gasmessvorrichtungen sind auch aus US 2002/079442 bekannt.

Die Grundlage der Gasabscheidung durch die Membran und deren Stützeinrichtung an der Gaseintrittsöffnung des Druckzylinders ist das Henry-Gesetz, demzufolge die Partialdrücke in der Gasphase den gelösten Konzentrationen in der Wasserphase entsprechen müssen. Dies gilt streng genommen, wenn sich ein Gleichgewicht eingestellt hat und die Membran keine unterschiedlichen Diffusionsbarrieren für verschiedene Gasspezies mit sich bringt. Letzteres muss untersucht und ggf. in der späteren Messauswertung berücksichtigt werden.

Das Einstellen des Gleichgewichts kann befördert werden durch das gezielte Anströmen der Gaseintrittsöffnung mit relativ geringen Volumenraten von einigen zehn Milliliter pro Sekunde. Dies hat den Effekt, die Ansprechzeit der Gasmessvorrichtung zu verringern. Wird die Gasmessvorrichtung indes an einem Tauchfahrzeug installiert, kann die Anströmung des Gaseinlasses durch die Fahrtbewegung bereits ausreichen, um die Ansprechzeit zu verkürzen. Es ist noch erwähnenswert, dass eine auf dem ICOS-Prinzip basierende Gasmessvorrichtung für den Unterwassereinsatz von Wankel et al., "Characterizing the Distribution of Methane Sources and Cycling in the Deep Sea via in Situ Stable Isotope Analysis", Environmental Science & Technology 2013 47 (3), 1478-1486 (DOI: 10.1021/es303661w) beschrieben wird, mit der sich neben der Konzentration von Methan auch das Verhältnis der stabilen Kohlenstoffisotope *δ*¹³*C*_{*CH*4} messen lässt, was Rückschlüsse auf die Methanquellen erlaubt.

Die beiden vorgenannten Druckschriften beschreiben Gasmessvorrichtungen, die eine Messkammer im Druckbehälter umfassen, die in Gas austauschender Kommunikation mit der (gestützten) Membran an der Gaseintrittsöffnung des Druckbehälters steht. Die Partialdrücke, die sich hinter der Membran - also innerhalb des Druckbehälters - durch Diffusion von Gasspezies aus dem Umgebungswasser einstellen, sollten in der Regel ohne nennenswerte zeitliche Verzögerung auch in der Messkammer vorliegen.

In der Messkammer können an sich beliebige Detektionseinrichtungen für physikalische und/oder chemische Parameter von Gas vorgesehen sein, in einem Fall ist es exemplarisch ein Massenspektrometer und im anderen sind eine Laserlichtquelle und ein Detektor am Ende eines durch Verspiegelung verlängerten Lichtpfades durch die Messkammer angeordnet. Weitere nützliche Detektionseinrichtungen sind Sensoren, die man auch zusätzlich vorsehen kann und die der Messung von Druck, Temperatur und Feuchte in der Messkammer dienen.

Detektionseinrichtungen geben üblich analoge elektrische Spannungen als Messwerte oder Ausgangssignale aus, die digitalisiert und elektronisch verarbeitet werden und typisch auch mit einem Zeitstempel versehen. In einigen Fällen beschreiben die digitalen Signale bereits direkt die gewünschten physikalischen und/oder chemischen Parameter des Gases in der Messkammer, in manchen Fällen muss eine elektronische Auswerteeinrichtung die Signale numerisch nachbearbeiten, z.B. verrechnen oder mit vorab tabellierten Daten vergleichen, um Derivatsignale zu bilden, die den Parametern entsprechen. Beispielsweise sind Laserlichtabsorptionsmesswerte durch eine Nachbearbeitung in Partialdruck- bzw. Konzentrationswerte umzurechnen. Es ist sinnvoll, wenigstens ein Signal oder ein Derivatsignal jeder Detektionseinrichtung als Repräsentanten eines erfassten Parameters von der Auswerteeinrichtung zu erzeugen und einer nicht-flüchtigen Datenspeicherung zuzuführen. Ist das Unterwasser-Gasmessgerät an einem Lander-System oder an einem Tauchroboter installiert, dann besteht oftmals eine Kabelverbindung zum Nutzer z.B. an Bord eines Schiffes. Die Messdaten können dann direkt zu einem protokollierenden Computer beim Nutzer weitergeleitet werden mittels digitaler Datenfernübertragung. Die Weiterleitung der Messdaten kann alternativ auch akustisch ohne Kabelverbindung erfolgen, wenn der empfangende Nutzer in Empfangsreichweite ist. Kompakte und kostengünstige nicht-flüchtige Datenspeicher oder Datenlogger sind überdies gängige eingebaute Standardkomponenten der meisten Unterwasser-Messvorrichtungen, weil oftmals keine direkte oder unverzügliche Datenübertragung zum Nutzer möglich ist.

Bemerkenswert ist, dass die Gasmessvorrichtungen der beiden genannten Druckschriften jeweils eine Vakuumpumpe zum Evakuieren der Messkammer aufweisen.

Das Absaugen einer Gasprobe aus der Messkammer verringert sowohl die Abklingzeit als auch die Ansprechzeit durch eine leicht erhöhte Diffusion durch die Membran. Überdies wird so vermieden, dass Reste der letzten Gasprobe erneut vermessen werden, es also zur fehlerhaften Korrelation aufeinanderfolgender Messungen kommt. Besonders bedeutsam ist dies, wenn die Gasmessvorrichtung zum Profilieren bewegt wird und dabei auf sehr unterschiedliche chemische Verhältnisse schon auf kurzen Strecken treffen kann, z.B. in der Nähe von Plumes.

Es ist auch bemerkenswert, dass die Autoren der Druckschriften nicht genau sagen, wohin die abgesaugte Gasprobe eigentlich verbracht wird. Dies ist ein wenig überraschend, denn man kann nicht vernünftigerweise unterstellen, dass die Vakuumpumpe oder irgendeine andere Gaspumpe problemlos in der Lage wäre, die nicht mehr benötigte Gasprobe gegen den Wasserdruck in der Tiefsee aus dem Druckzylinder auszublasen. Im Übrigen würde ein solches Ausblasen erhebliche mechanische Arbeit verrichten müssen und somit die interne Energieversorgung der Gasmessvorrichtung sehr belasten. Ein Hinweis auf die Entsorgung der gemessenen Gasprobe in die Umgebung ist jedenfalls weder der US 2009/0084976 A1 noch dem Paper von Wankel et al. zu entnehmen.

Dies rechtfertigt die Annahme, dass die Gasprobe aus dem Messkreis in eine Auffangkammer im Innern des Druckbehälters gepumpt wird. Im Extremfall kann dabei der gesamte innere Bauraum des Druckbehälters, der nicht von Komponenten der Gasmessvorrichtung belegt ist, als Auffangkammer dienen. Der Stand der Technik legt somit den folgenden Aufbau der Absaugeinrichtung nahe, der der Messkammer mit Gasauslass nachgeschaltet ist:
a. eine Vakuumpumpe;
b. ein der Vakuumpumpe nachgeschaltetes Rückschlagventil angeordnet in Durchlassrichtung;
c. eine dem Rückschlagventil nachgeschaltete Auffangkammer für gemessenes Gas;
d. eine Pumpenansteuereinrichtung, die dazu ausgebildet ist, die Vakuumpumpe zu veranlassen, Gas aus der Messkammer abzusaugen und der Auffangkammer zuzuführen.

Das Errichten des Vakuums bzw. Unterdrucks in der Messkammer kann sensorisch erfasst werden. Die Vakuumpumpe ist dazu ausgelegt, die Messkammer in wenigen Sekunden weitgehend zu entleeren, wohingegen die Diffusion durch die Membran deutlich langsamer abläuft. Die Pumpe kann dabei auch gegen einen - begrenzten - Überdruck arbeiten, und in jedem Fall wird der Gasdruck in der Auffangkammer mit der Anzahl der Messungen, also der steigenden Anzahl abgesaugter Gasproben, ansteigen. Der Pumpe wird daher naheliegend ein Rückschlagventil in Durchlassrichtung nachgeschaltet, das Gas nur vom Pumpenausgang in die Auffangkammer strömen lässt, bei abgeschalteter Pumpe aber gegen den Überdruck in der Auffangkammer selbsttätig schließt.

Die US 2009/0084976 A1 sagt in Abs. 0004 aus: "[...] there is a need for a submersible system to perform long-term series sampling of dissolved gases in a water column in the ocean depths (e.g., at depths greater than 2500 m)." Hier stellt sich die Frage, wie viele Messungen man dafür als ausreichend ansehen sollte. Zahlreiche am Meeresboden verankerte Aggregate werden während einer Messkampagne mit einem Schiff ausgesetzt und erst Monate oder gar ein Jahr später wieder geborgen. An interessanten Einsatzorten - etwa an natürlichen Gasquellen wie z.B. "schwarzen Rauchern" - wird man mit einer oder zwei Messungen pro Tag der Zielsetzung nicht gerecht. Vielmehr sollte man die Größenordnung von mehreren Tausend Messungen während eines Einsatzes ins Auge fassen. Auch für das Profilieren von Gasverteilungen mit Tauchfahrzeugen wäre diese Größenordnung sehr wünschenswert, da hierbei möglichst alle paar Sekunden eine Gasmessung erfolgen sollte.

Spätestens dann, wenn die Zahl der abgesaugten Gasproben so groß wird, dass der Gasdruck in der Auffangkammer die Leistungsfähigkeit der Vakuumpumpe übersteigt, sind keine weiteren Messungen mehr ausführbar.

Die Erfindung stellt sich die Aufgabe, eine Unterwasser-Gasmessvorrichtung vorzuschlagen, die zur Durchführung einer sehr großen Anzahl von Messungen besser geeignet ist als die vorbekannten Messvorrichtungen.

Die Aufgabe wird gelöst durch eine Unterwasser-Gasmessvorrichtung für in Wasser gelöste Gase aufweisend
a. einen Druckbehälter mit einer Gaseintrittsöffnung in der Wand des Druckbehälters;
b. eine semipermeable, für Gas durchlässige Membran wasserdicht abschließend angeordnet an der Gaseintrittsöffnung;
c. eine Stützeinrichtung für die Membran gegen hydrostatischen Druck;
d. eine Messkammer im Druckbehälter in Gas austauschender Kommunikation mit der Membran und aufweisend einen Gasauslass;
e. eine Detektionseinrichtung zur Erfassung wenigstens eines physikalischen und/oder chemischen Parameters von Gas in der Messkammer;
f. eine elektronische Auswerteeinrichtung ausgebildet zur Erfassung wenigstens eines Signals der Detektionseinrichtung repräsentierend wenigstens einen erfassten Parameter und ausgebildet zur digitalisierten Weiterleitung und/oder zur nicht-flüchtigen digitalen Speicherung des wenigstens einen Signals und/oder wenigstens eines Derivatsignals;
g. eine dem Gasauslass der Messkammer nachgeschaltete Vakuumpumpe;
h. ein der Vakuumpumpe nachgeschaltetes Rückschlagventil angeordnet in Durchlassrichtung;
i. eine dem Rückschlagventil nachgeschaltete Auffangkammer für gemessenes Gas;
j. eine Pumpenansteuereinrichtung, die dazu ausgebildet ist, die Vakuumpumpe zu veranlassen, Gas aus der Messkammer abzusaugen und der Auffangkammer zuzuführen; dadurch gekennzeichnet, dass
k. die Auffangkammer ein veränderliches Volumen und einen Gasauslass aufweist;
l. ein Überdruckventil am Gasauslass der Auffangkammer angeordnet ist, das bei Überschreiten eines vorbestimmten Gasdrucks in der Auffangkammer öffnet;
m. mechanische Mittel zur Volumenänderung der Auffangkammer vorgesehen sind, die bei Gaszufuhr das Vergrößern des Volumens der Auffangkammer bis zu einem vorbestimmten Maximalvolumen unter Beibehaltung des vorbestimmten Gasdrucks zulassen;
n. ein Antrieb für die mechanischen Mittel zur Volumenänderung vorgesehen und dazu ausgebildet ist, die mechanischen Mittel bei Erreichen des vorbestimmten Maximalvolumens zum Verkleinern des Volumens der Auffangkammer unter Ausblasen des Gasinhalts durch das Überdruckventil zu veranlassen, wobei
o. eine Gasleitung das Gas vom Ausgang des Überdruckventils zu einer Gasaustrittsöffnung in der Wand des Druckbehälters führt.

Die Unteransprüche geben vorteilhafte Ausgestaltungen an.

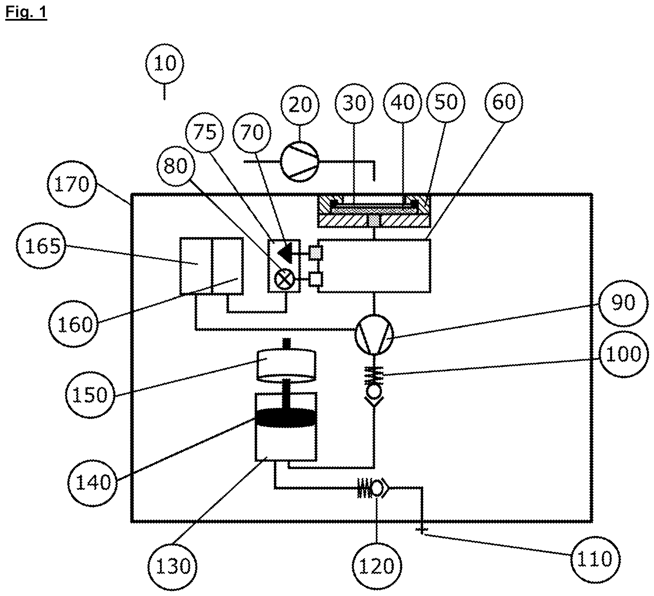

Zur näheren Erläuterung der Erfindung dient auch die einzige Fig. 1, die eine Skizze der Unterwasser-Gasmessvorrichtung mit darin bezeichneten Komponenten zeigt. Die bereits aus dem Stand der Technik vorbekannten Komponenten sind im Einzelnen:
- 10: Umgebungswasser
- 20: Anströmpumpe
- 30: Membran
- 40: Stützeinrichtung für die Membran, z.B. Metallscheibe
- 50: Membranhalterung
- 60: Messkammer
- 75: Detektionseinrichtung (z.B. Lichtquelle 70 und Lichtdetektor 80)
- 90: Vakuumpumpe
- 100: Rückschlagventil
- 130: Auffangkammer
- 160/165: Auswerteeinrichtung / Pumpenansteuereinrichtung (Prozessor)
- 170: Druckbehälter (mit Gaseintrittsöffnung oben)

Die erfindungsgemäße Ausgestaltung des Unterwasser-Gasmessgerätes weist eine Auffangkammer 130 auf, die auch während des sukzessiven Befüllens mit abgesaugten Gasproben stets einen konstanten Gasdruck im Innern beibehält. Die Auffangkammer 130 wird daher beim Befüllen mit Gas expandiert, d.h. ihr Volumen vergrößert sich. Erreicht die Auffangkammer 130 ein vorbestimmtes Maximalvolumen, dann wird ein Antrieb 150 aktiviert, der die Auffangkammer 130 durch mechanische Krafteinwirkung wieder komprimiert. Das enthaltene Gas wird dabei aus der Auffangkammer 130 ausgeblasen, und zwar durch eine Gasleitung, die zu einer Gasaustrittsöffnung 110 in der Wand des Druckbehälters 170 führt.

Unter Druckbedingungen der Tiefsee soll das Ausblasen nicht in das Umgebungswasser 10 erfolgen, sondern in einen separaten Drucktank (nicht dargestellt), der eigens zur Aufnahme der Gasproben mitgeführt wird. Der Drucktank ist über eine druckbeständige Gasleitung, vorzugsweise ein Rohr aus Edelstahl, mit dem Gasauslass der Gasmessvorrichtung verbunden. Die Gasleitung ist an beiden Enden wasserdicht angeflanscht, und insbesondere ist dadurch die Gasaustrittsöffnung 110 der Gasmessvorrichtung vom Umgebungsdruck entlastet.

Der Drucktank kann ein vergleichsweise einfaches, druckstabiles Behältnis sein, beispielsweise eine geschlossene Hohlkugel oder ein geschlossener Hohlzylinder mit halbkugelförmigen Deckkappen, und muss lediglich einen Gaseinlass und einen Gasauslass aufweisen. Es können auch mehrere Drucktanks zusammengeschaltet sein, so dass etwa der Gasauslass eines ersten Drucktanks mittels einer druckbeständigen Gasleitung mit dem Gaseinlass eines zweiten Drucktanks verbunden ist. Diese Gasleitung kann dabei dauerhaft geöffnet sein, so dass es jederzeit zum Ausgleich des Gasdrucks zwischen den verschiedenen Drucktanks kommt. Wenigstens ein Drucktank wird einen durch ein geschlossenes Ventil geschlossenen Gasauslass aufweisen. Vorzugsweise gestattet dieses Ventil das selbsttätige Öffnen des Gasauslasses, sobald der Umgebungsdruck unter den Gasdruck im Drucktank sinkt. Dies erfolgt wenigstens beim Einbringen der Gasmessvorrichtung, wenn diese zum Meeresspiegel angehoben wird. Es wird als sicherer angesehen, das nicht mehr benötigte Gas, das im Drucktank unter Überdruck steht, automatisch abzulassen, bevor die Messvorrichtung ein Schiffsdeck mit Personen erreicht.

Die erfindungsgemäße Unterwasser-Gasmessvorrichtung weist also bevorzugt wenigstens einen der Gasaustrittsöffnung 110 nachgeschalteten Drucktank mit einem Gasauslass in die Umgebung auf, wobei der Gasauslass öffnet, sobald der Innendruck des Drucktank den Umgebungsdruck übersteigt.

Das Anschließen von Drucktanks an die Gasmessvorrichtung ist als optional anzusehen. Wünscht man die Gasmessvorrichtung nur bei wenigen zehn Metern Wassertiefe einzusetzen, etwa in der Ostsee, dann kann das Ausblasen der Auffangkammer ohne weiteres in das Umgebungswasser 10 erfolgen. In diesem Fall wäre die Anzahl der durchführbaren Gasmessungen allein durch die verfügbare Energie, aber nicht mehr durch das Auffangkammervolumen begrenzt.

In der Tiefsee kann man die Zahl der möglichen Messungen durch die Wahl von Anzahl und Größe der Drucktanks zumindest in gewissen Grenzen steuern. Es spricht auch nichts dagegen, den wenigstens einen Drucktank zu evakuieren, bevor er an der Gasmessvorrichtung angeflanscht und mit dieser zusammen zu Wasser gelassen wird.

Es wird als Vorteil der Erfindung angesehen, dass dasselbe Gerät ohne Umbau der inneren Messgeräte und Installationen für Flachwasser- und Tiefseemessungen geeignet ist. Es wird als ein weiterer Vorteil angesehen, dass die Vakuumpumpe 90 jederzeit gegen einen konstanten vorbestimmten Gasdruck anpumpt, wenn sie eine gemessene Gasprobe absaugt und der Auffangkammer 130 zuführt. Die macht insbesondere den Energiebedarf der Pumpe 90 pro Messung gut kalkulierbar.

Die Auffangkammer 130 ist erfindungsgemäß als ein mechanischer Gasverdichter ausgebildet.

Eine mögliche, aber nicht in Fig. 1 skizzierte Ausgestaltung kann in einem flexiblen Ballon, z.B. aus einer Kunststofffolie, bestehen, die zwischen zwei parallelen, starren Platten, z.B. aus Metall, angeordnet ist. Beim Befüllen mit Gas dehnt sich der Ballon aus und drückt die Platten auseinander. Beim Erreichen eines Maximalvolumens, z.B. korrespondierend zu einem Maximalabstand der Platten, wird der Antrieb, vorzugsweise ein Elektromotor, aktiviert, der die Platten wieder zusammendrückt und so das Volumen des Ballons verringert. Anders gesagt kann der Gasverdichter nach Art eines Blasebalgs funktionieren.

Besonders bevorzugt kann man wie in Fig. 1 einen Kolbenverdichter in Betracht ziehen, dessen Innenraum die Auffangkammer 130 bilden soll. In einer typischen Bauweise ist der Kolbenverdichter ein zylindrisches, einseitig gedeckeltes Rohr, in dessen offenes Ende ein luftdicht mit der Rohrwandung abschließender Kolben 140 eingeführt ist. Weist die gedeckelte Seite des Rohres wenigstens eine Öffnung auf, die als Gaseinlass oder Gasauslass oder beides dient, kann der Kolben durch mechanische Bewegung Gas in den Innenraum 130 einsaugen oder ausblasen - nach demselben Prinzip wie eine Fahrrad-Luftpumpe.

Für die Zwecke der Erfindung kann es besonders vorteilhaft sein, den Innenraum 130 eines Kolbenverdichters als Auffangkammer zu verwenden, wenn dabei der Kolbenverdichter vertikal angeordnet ist, wobei der Gasauslass an der Unterseite des Verdichters angeordnet ist und der Kolben 140 von oben auf das Gas in der Auffangkammer 130 drückt. Dabei kann der Gasauslass zugleich auch als Gaseinlass für Gas von der Vakuumpumpe 90 kommend dienen. Besonders bevorzugt ist nämlich der Kolben 140 dann frei beweglich im Verdichter gelagert und bestimmt unter Schwerkraftwirkung den Druck in der Auffangkammer 130. Führt die Vakuumpumpe 90 der Auffangkammer 130 dann eine weitere gemessene Gasprobe zu, arbeitet sie gegen das konstante Gewicht des Kolbens 140 und hebt diesen ein wenig an. Es liegt der stets gleiche Gasdruck in der Auffangkammer 130 vor, bei dem das Überdruckventil 120 am Gasauslass der Auffangkammer 130 geschlossen bleibt. Der Kolben 140 kann bis zu einer vorbestimmten Höhe angehoben werden, die zugleich das Maximalvolumen der Auffangkammer 130 festlegt. Vorzugsweise weist der Kolbenverdichter ein Schaltelement (nicht dargestellt) auf, das betätigt wird, wenn der Innenraum 130 des Kolbenverdichters das vorbestimmte Maximalvolumen annimmt, und das den Antrieb 150 zur Kraftausübung auf den Kolben 140 in Richtung auf den Gasauslass veranlasst. Der Antrieb 150 kann auch hier ein Elektromotor sein, der direkt auf den Kolben 140 einwirkt. Das Schaltelement kann beispielsweise ein mechanisches Element sein, das oberhalb des Kolbenkopfes im Verdichter, also außerhalb der Auffangkammer 130, angeordnet ist. Wird der Kolbenkopf über eine vorbestimmte Höhe angehoben, übt er eine Kraft auf den Schalter aus, der dadurch betätigt wird. Das Betätigen des Schalters aktiviert den Antrieb 150 zur Krafteinwirkung auf den Kolben 140. Vorzugsweise springt das mechanische Schaltelement bei Entlastung wieder in seine Ausgangsposition zurück, was aber nicht den Antrieb 150 deaktivieren muss.

Schon eine relativ geringe Kraft führt zu einer Kompression des unter dem Kolben 140 in der Auffangkammer 130 befindlichen Gasvolumens und erhöht dort den Gasdruck, wodurch das Überdruckventil 120 öffnet und sich die Auffangkammer 130 durch den Gasausfluss entleert. Das Gas kann gegebenenfalls in den wenigstens einen Drucktank abfließen, bis der Kolben 140 einen mechanischen Anschlag erreicht. Bei Erreichen des Anschlages kann der Antrieb 150 deaktiviert und in seinen Ausgangszustand gebracht werden. Insbesondere ist der Schwerkraftkontrollierte Kolben 140 dann wieder frei beweglich. Das Überdruckventil 120 schließt nach Abbau des Überdrucks selbsttätig, und die Auffangkammer 130 hat ihr kleinstes Volumen angenommen. Weiteres Gas kann von der Vakuumpumpe 90 unter denselben Bedingungen wie zuvor zugeführt werden.

Es wird als vorteilhaft angesehen, wenn die Vakuumpumpe 90 dazu ausgebildet ist, in der Messkammer 60 einen Unterdruck von höchstens 100 hPa (= 0,1 Atmosphären = 0,1 bar) gegenüber einem ausgangsseitigen Gasdruck von wenigstens 0,3 MPa (= 3 Atmosphären = 3 bar) in wenigen Sekunden herzustellen. Es wird weiterhin als vorteilhaft betrachtet, dass der Gasdruck in der Auffangkammer 130 wenigstens 0,3 MPa beträgt. Je höher der Gasdruck vorbestimmt wird, desto größer ist die effektive Kompression der abgesaugten Gasprobe und desto mehr Messungen sind bei dem vorbestimmten Gesamtvolumen von Drucktanks ausführbar.

Wenn die Auffangkammer ein zylindrischer Kolbenverdichter mit 2 cm Innendurchmesser ist, dann benötigt der frei bewegliche Kolben 140 eine Masse von fast 10 kg, um allein durch sein Gewicht einen Gasdruck von 0,3 MPa in der Auffangkammer 130 herzustellen. Ein zu hohes Gesamtgewicht der Gasmessvorrichtung ist allerdings schon aus Gründen des Handlings an Bord nicht wünschenswert.

Alternativ kann der Antrieb 150 des Kolbens 140 - oder auch irgendeiner anderen baulichen Ausgestaltung des Gasverdichters - so geregelt werden, dass er den vorbestimmten Gasdruck durch aktive Krafteinwirkung in der Auffangkammer 130 stets konstant hält. Hierfür kann ein zusätzlicher Drucksensor in der Auffangkammer 130 zweckmäßig sein, dessen Messwerte der Ansteuerung des Antriebs 150 - in der Regel eine elektronische Prozessoreinheit - zugeführt werden.

In einer beispielhaften Ausführungsform ist die Unterwasser-Gasmessvorrichtung an einer Plattform installiert, die auf eine gewünschte Messtiefe abgesenkt werden kann und eine Stromversorgung für verschiedene Messgeräte bereitstellt.

Wie bereits erwähnt wird die Diffusion des in Umgebungswasser gelösten Gases durch die Membran 30 an der Gaseintrittsöffnung der Gasmessvorrichtung dadurch beschleunigt, dass eine Anströmung der Membran 30 mit einer Anströmpumpe 20 erfolgt. Die Anströmpumpe 20 kann dabei als ein fest integrierter Bestandteil der Gasmessvorrichtung ausgestaltet sein, die dann durch die Gasmessvorrichtung mit Energie versorgt wird.

Das Einstellen eines Gleichgewichts der Gaskonzentration in der Messkammer 60 gegenüber dem Umgebungswasser 10 gehorcht dem Fick'schen Diffusionsgesetz, d.h. die Gaskonzentration folgt einer Exponentialfunktion der Zeit, und die Berechnung des Endwerts kann aus dem Kurvenverlauf erfolgen. Es ist somit nicht unbedingt erforderlich, die t₉₀-Messzeit zur Ermittlung eines Messwertes abzuwarten. Der Nutzer kann die tatsächliche Messzeit deutlich reduzieren, wenn er dafür eine höhere Messungenauigkeit akzeptiert.

Die Vakuumpumpe 90 besitzt im Ausführungsbeispiel eine Förderleistung größer als 15 Liter/min und erzeugt ein Vakuum - einen Unterdruck von unter 100 hPa - in einem Gasvolumen von insgesamt 25 Milliliter umfassend Membranhalterung 50 und Messkammer 60 in weniger als 5 Sekunden. Aufgrund des geringen Gasvolumens reduziert sich die Zeit bis zum Einstellen des Gleichgewichts in der Messzelle 60 durch das Evakuieren auf t₉₀ < 15 s.

Nachdem ein Messwert von der Detektionseinrichtung 75 - im Beispiel mit einer optischen Messung unter Verwendung einer Lichtquelle 70 und eines Lichtdetektors 80 - bestimmt und von der elektronischen Auswerteeinrichtung 160 verarbeitet worden ist, aktiviert die Pumpenansteuereinrichtung 165 die Vakuumpumpe 90, um das gemessene Gasvolumen von der Messkammer 60 zur Auffangkammer 130 zu übertragen. Die Auffangkammer 130 kann mit zusätzlichen Drucksensoren (nicht dargestellt) ausgestattet sein. Wie bereits beschrieben, wird die Auffangkammer 130 dabei expandiert, wobei der Druck in der Auffangkammer 130 konstant zu halten ist.

In der Fig. 1 sind die Auswerteeinrichtung 160 und die Pumpenansteuereinrichtung 165 als eine bauliche Einheit dargestellt. Dies muss nicht so sein, ist aber zweckmäßig, da die Pumpenansteuereinrichtung 165 erst dann die Vakuumpumpe 90 aktivieren soll, wenn die Messung eines Gasvolumens beendet und die Messdaten von der Auswerteeinrichtung 160 verarbeitet, d.h. z.B. im internen Datenlogger aufgezeichnet oder per Kabel an den Anwender übertragen, worden sind. Die beiden Einrichtungen müssen also miteinander kommunizieren und können üblich als herkömmliche Computer-Prozessoren mit Software ausgebildet sein. Es ist unproblematisch, die Einrichtungen 160 und 165 mit einem einzigen Prozessor zu realisieren.

Im Ausführungsbeispiel ist die Auffangkammer 130 - wie oben beschrieben - der Innenraum eines Kolbenverdichters mit Maximalvolumen 20 Milliliter. Während der Gasaufnahme wird die Auffangkammer 130 bei einem konstanten Druck von 0,4 MPa gehalten. Die Vakuumpumpe 90 leistet beim Überführen der gemessenen Gasprobe von der Messkammer 60 in die Auffangkammer 130 eine erste Verdichtung, d.h. die Gasprobe wird durch den Druckanstieg auf ihrem Weg komprimiert. Das Volumen der Auffangkammer 130 erhöht sich durch die Aufnahme einer einzelnen Gasprobe um deutlich weniger als 25 Milliliter, typisch um ungefähr 5-6 Milliliter. Die Vakuumpumpe 90 im Ausführungsbeispiel ist so ausgelegt, dass sie auf einen Ausgangsdruck von bis zu 0,7 MPa verdichten kann.

Der Kolbenverdichter des Ausführungsbeispiels ist - nunmehr alternativ zum Schwerkraftkontrollierten Kolbenverdichter - mit einem starken Antrieb 150 ausgestattet, der im Innenraum 130 einen Gasdruck von bis zu 5 MPa aufbauen kann (zweite Verdichtung). Der tatsächlich erreichte Maximaldruck im Verdichter hängt von der Auslegung des Überdruckventils 120 am Gasauslass der Auffangkammer ab. Das Überdruckventil 120 kann dazu ausgelegt sein, erst bei einem Gasdruck um 5 MPa zu öffnen.

Die beispielhafte Gasmessvorrichtung unterliegt somit keiner Begrenzung für die maximale Anzahl von Messungen bis zu einer Messtiefe von ungefähr 500 m, da das gemessene Gas zunächst in die Auffangkammer 130 transferiert und anschließend in das Umgebungswasser 10 durch die Gasaustrittsöffnung 110 ausgeblasen werden kann. Wie bereits erwähnt, ist der Energiebedarf für den Kolbenverdichter nicht unerheblich, so dass eine direkte Stromversorgung vom Forschungsschiff wünschenswert ist.

Bei größeren Messtiefen, die kein Ausblasen des Gases in die Umgebung 10 mehr zulassen, ist das Anschließen wenigstens eines externen Tanks vorgesehen. Der beispielhafte externe Tank hat ein Innenvolumen von 500 Milliliter, was die Möglichkeit zur Aufnahme von mindestens eintausend (1000) Gasproben bietet, denn der Kolbenverdichter kann die ursprünglichen 25 Milliliter Gasvolumen einer Gasprobe auf höchstens 0,5 Milliliter im externen Tank komprimieren. Der Tank kann einem maximalen Innendruck von 5 MPa und einem Außendruck von 60 MPa standhalten.

Die vorgeschlagene Erfindung ermöglicht den Bau einer Gasmessvorrichtung, mit der man sehr schnell und eine große Anzahl von Konzentrationsdaten im Umgebungswasser 10 ermitteln kann, und sie eröffnet die Möglichkeit, sehr genaue Konzentrationsprofile verschiedener Gase gleichzeitig zu erstellen.

## Patentansprüche

1. Unterwasser-Gasmessvorrichtung für in Wasser gelöste Gase aufweisend
a. einen Druckbehälter (170) mit einer Gaseintrittsöffnung in der Wand des Druckbehälters (170),
b. eine semipermeable, für Gas durchlässige Membran (30) wasserdicht abschließend angeordnet an der Gaseintrittsöffnung;
c. eine Stützeinrichtung (40) für die Membran (30) gegen hydrostatischen Druck;
d. eine Messkammer (60) im Druckbehälter in Gas austauschender Kommunikation mit der Membran (30) und aufweisend einen Gasauslass;
e. eine Detektionseinrichtung (75) zur Erfassung wenigstens eines physikalischen und/oder chemischen Parameters von Gas in der Messkammer (60);
f. eine elektronische Auswerteeinrichtung (160) ausgebildet zur Erfassung wenigstens eines Signals der Detektionseinrichtung (75) repräsentierend wenigstens einen erfassten Parameter und ausgebildet zur digitalisierten Weiterleitung und/oder zur nicht-flüchtigen digitalen Speicherung des wenigstens einen Signals und/oder wenigstens eines Derivatsignals;
g. eine dem Gasauslass der Messkammer (60) nachgeschaltete Vakuumpumpe (90);
h. ein der Vakuumpumpe (90) nachgeschaltetes Rückschlagventil (100) angeordnet in Durchlassrichtung;
i. eine dem Rückschlagventil (100) nachgeschaltete Auffangkammer (130) für gemessenes Gas;
j. eine Pumpenansteuereinrichtung (165), die dazu ausgebildet ist, die Vakuumpumpe (90) zu veranlassen, Gas aus der Messkammer (30) abzusaugen und der Auffangkammer (130) zuzuführen;
**dadurch gekennzeichnet, dass**
k. die Auffangkammer (130) ein veränderliches Volumen und einen Gasauslass aufweist;
l. ein Überdruckventil (120) am Gasauslass der Auffangkammer (130) angeordnet ist, das bei Überschreiten eines vorbestimmten Gasdrucks in der Auffangkammer (130) öffnet;
m. mechanische Mittel (140) zur Volumenänderung der Auffangkammer (130) vorgesehen sind, die bei Gaszufuhr das Vergrößern des Volumens der Auffangkammer (130) bis zu einem vorbestimmten Maximalvolumen unter Beibehaltung des vorbestimmten Gasdrucks zulassen;
n. ein Antrieb (150) für die mechanischen Mittel (140) zur Volumenänderung vorgesehen und dazu ausgebildet ist, die mechanischen Mittel (140) bei Erreichen des vorbestimmten Maximalvolumens zum Verkleinern des Volumens der Auffangkammer (130) unter Ausblasen des Gasinhalts durch das Überdruckventil (120) zu veranlassen, wobei
o. eine Gasleitung das Gas vom Ausgang des Überdruckventils (120) zu einer Gasaustrittsöffnung (110) in der Wand des Druckbehälters (170) führt.

2. Vorrichtung nach Anspruch 1,
**gekennzeichnet durch**
wenigstens einen der Gasaustrittsöffnung (110) nachgeschalteten Drucktank mit einem Gasauslass in die Umgebung, wobei der Gasauslass öffnet, sobald der Innendruck des Drucktank den Umgebungsdruck übersteigt.

3. Vorrichtung nach einem der vorangehenden Ansprüche,
**dadurch gekennzeichnet, dass**
die Auffangkammer (130) als Innenraum eines Kolbenverdichters ausgebildet ist.

4. Vorrichtung nach Anspruch 3,
**dadurch gekennzeichnet, dass**
der Kolbenverdichter vertikal angeordnet ist, wobei der Gasauslass an der Unterseite des Verdichters angeordnet ist und der Kolben (140) von oben auf das Gas in der Auffangkammer (130) drückt.

5. Vorrichtung nach Anspruch 4,
**dadurch gekennzeichnet, dass**
der Kolben (140) frei beweglich im Verdichter gelagert ist und unter Schwerkraftwirkung den Druck in der Auffangkammer (130) bestimmt.

6. Vorrichtung nach einem der Ansprüche 3 bis 5,
**dadurch gekennzeichnet, dass**
der Kolbenverdichter ein Schaltelement aufweist, das betätigt wird, wenn der Innenraum des Kolbenverdichters ein vorbestimmtes Maximalvolumen annimmt, und das den Antrieb (150) zur Kraftausübung auf den Kolben (140) in Richtung auf den Gasauslass veranlasst.

7. Vorrichtung nach einem der vorangehenden Ansprüche,
**dadurch gekennzeichnet, dass**
der vorbestimmte Gasdruck in der Auffangkammer (130) wenigstens 3 bar (0,3 MPa) beträgt.

## Claims

1. An underwater gas measuring device for gases dissolved in water comprising
a. a pressure vessel (170) with a gas inlet opening in the wall of the pressure vessel (170)
b. a semi-permeable, gas-permeable membrane (30) arranged at the gas inlet opening in a watertight manner;
c. a support device (40) for the membrane (30) against hydrostatic pressure;
d. a measuring chamber (60) in the pressure vessel in gas exchanging communication with the membrane (30) and having a gas outlet;
e. a detection device (75) for detecting at least one physical and/or chemical parameter of gas in the measuring chamber (60);
f. an electronic evaluation device (160) designed to detect at least one signal of the detection device (75) representing at least one detected parameter and designed for digitized transmission and/or for non-volatile digital storage of at least one signal and/or at least one derivative signal;
g. a vacuum pump (90) connected downstream of the gas outlet of the measuring chamber (60);
h. a non-return valve (100) connected downstream of the vacuum pump (90) and arranged in the flow direction;
i. a collecting chamber (130) downstream of the non-return valve (100) for measured gas;
j. a pump control device (165) which is designed to cause the vacuum pump (90) to draw gas from the measuring chamber (30) and to supply it to the collecting chamber (130);
**characterized in that**
k. the collection chamber (130) has a variable volume and a gas outlet;
l. a pressure relief valve (120) is arranged at the gas outlet of the receiving chamber (130), which opens when a predetermined gas pressure in the receiving chamber (130) is exceeded;
m. mechanical means (140) are provided for changing the volume of the receiving chamber (130) which, when gas is supplied, allow the volume of the receiving chamber (130) to be increased up to a predetermined maximum volume while maintaining the predetermined gas pressure;
n. a drive (150) is provided for the mechanical means (140) for changing the volume and is designed to cause the mechanical means (140), when the predetermined maximum volume is reached, to reduce the volume of the collecting chamber (130) while blowing out the gas content through the pressure relief valve (120), where
o. a gas pipe leads the gas from the outlet of the pressure relief valve (120) to a gas outlet opening (110) in the wall of the pressure vessel (170).

2. Device according to claim 1,
**characterised by**
at least one pressure tank downstream of the gas outlet opening (110) with a gas outlet to the environment, the gas outlet opening as soon as the internal pressure of the pressure tank exceeds the ambient pressure.

3. Device according to one of the preceding claims,
**characterised in that**
the collecting chamber (130) is designed as the interior of a piston compressor.

4. Device according to claim 3,
**characterised in that**
the piston compressor is arranged vertically, the gas outlet being arranged at the bottom of the compressor and the piston (140) pressing on the gas in the collecting chamber (130) from above.

5. Device according to claim 4,
**characterised in that**
the piston (140) is mounted freely movable in the compressor and determines the pressure in the collecting chamber (130) under the effect of gravity.

6. Device according to one of the preceding claims 3 to 5,
**characterised in that**
the piston compressor has a switching element which is actuated when the interior of the piston compressor assumes a predetermined maximum volume and which causes the drive (150) to exert force on the piston (140) in the direction of the gas outlet.

7. Device according to one of the preceding claims,
**characterised in that**
the predetermined gas pressure in the receiving chamber (130) is at least 3 bar (0.3 MPa).

## Revendications

1. Dispositif sous-marin de mesure des gaz dissous dans l'eau comprenant
a. un réservoir sous pression (170) avec une ouverture d'entrée de gaz dans la paroi du réservoir sous pression (170),
b. une membrane semi-perméable, perméable au gaz (30) disposée de manière hermétique à l'eau sur l'ouverture d'entrée de gaz ;
c. un dispositif de support (40) pour la membrane (30) contre la pression hydrostatique ;
d. une chambre de mesure (60) dans le réservoir sous pression en communication d'échange de gaz avec la membrane (30) et comprenant une sortie de gaz ;
e. un dispositif de détection (75) pour détecter au moins un paramètre physique et/ou chimique du gaz dans la chambre de mesure (60) ;
f. un dispositif d'évaluation électronique (160) conçu pour détecter au moins d'un signal du dispositif de détection (75) représentant au moins un paramètre détecté et conçu pour la transmission numérique et/ou pour la mémorisation numérique non volatile du au moins un signal et/ou d'au moins un signal dérivé ;
g. une pompe à vide (90) connectée en aval de la sortie de gaz de la chambre de mesure (60) ;
h. un clapet anti-retour (100) connecté en aval de la pompe à vide (90) et disposé dans le sens de l'écoulement ;
i. une chambre de collecte (130) pour le gaz mesuré, placée en aval du clapet anti-retour (100);
j. un dispositif de commande de la pompe (165) conçu pour amener la pompe à vide (90) à aspirer le gaz de la chambre de mesure (30) et à le conduire à la chambre de collecte (130) ;
**caractérisé en ce que**
k. la chambre de collecte (130) a un volume variable et une sortie de gaz ;
l. une valve de surpression (120) est disposée à la sortie de gaz de la chambre de collecte (130), qui s'ouvre lorsqu'une pression de gaz prédéterminée est dépassée dans la chambre de collecte (130) ;
m. des moyens mécaniques (140) sont prévus pour modifier le volume de la chambre de collecte (130) qui, lorsque du gaz est fourni, permettent d'augmenter le volume de la chambre de collecte (130) jusqu'à un volume maximal prédéterminé tout en maintenant la pression de gaz prédéterminée ;
n. un entraînement (150) est prévu pour les moyens mécaniques (140) de modification du volume et est conçu pour amener les moyens mécaniques (140), lorsque le volume maximal prédéterminé est atteint, à réduire le volume de la chambre de collecte (130) tout en évacuant le gaz contenu par la soupape de décompression (120), où
o. un tuyau de gaz conduit le gaz de la sortie de la soupape de décompression (120) à une ouverture de sortie de gaz (110) dans la paroi du réservoir sous pression (170).

2. Dispositif selon la revendication 1,
**caractérisé par**
au moins un réservoir sous pression placé en aval de l'ouverture de sortie du gaz (110) et doté d'une sortie de gaz vers l'environnement, la sortie de gaz s'ouvre dès que la pression interne du réservoir sous pression dépasse la pression ambiante.

3. Dispositif selon l'une des revendications précédentes,
**caractérisé en ce que**
la chambre de collecte (130) est conçue comme l'intérieur d'un compresseur à piston.

4. Dispositif selon la revendication 3,
**caractérisé en ce que**
le compresseur à piston est disposé verticalement, la sortie de gaz étant disposée au bas du compresseur et le piston (140) exerçant une pression sur le gaz dans la chambre de collecte (130) par le haut.

5. Dispositif selon la revendication 4,
**caractérisé en ce que**
le piston (140) est monté librement mobile dans le compresseur et détermine la pression dans la chambre de collecte (130) sous l'effet de la gravité.

6. Dispositif selon l'une des revendications précédentes 3 à 5,
**caractérisé en ce que**
le compresseur à piston comporte un élément de commutation qui est actionné lorsque l'intérieur du compresseur à piston atteint un volume maximal prédéterminé et qui amène l'entraînement (150) à exercer une force sur le piston (140) en direction de la sortie de gaz.

7. Dispositif selon l'une des revendications précédentes,
**caractérisé en ce que**
la pression de gaz prédéterminée dans la chambre de collecte (130) est d'au moins 3 bars (0,3 MPa).
